# EUROPEAN PATENT APPLICATION

(11) **EP 2 333 104 A1**
(43) Date of publication of application: **15.06.2011**
(21) Application number: 09178923.0
(22) Date of filing: 11.12.2009
(51) Int. Cl.: C12Q 1/68

(54) **RNA analytics method**

(71) Applicant: Lexogen GmbH, 1230 Vienna (AT)
(72) Inventor: Seitz, Alexander, 1140 Vienna (AT); Paul, Lukas, 1230 Vienna (AT); van Min, Max Jan, 2497 ZJ Den Haag (NL)
(74) Representative: Sonn & Partner Patentanwälte

(57) **Abstract**

The present invention relates to a method of ordering nucleic acid molecule fragment sequences derived from a pool of potentially diverse RNA molecules comprising
• optionally reverse transcribing the RNA molecules to provide a pool of cDNA molecules,
• segregating nucleic acids from said template RNA or cDNA pool, selecting for potentially different templates with a distinctive nucleic acid feature shared by the segregated templates, thereby providing at least a first subpool of nucleic acids,
• optionally once or more further segregating nucleic acids from said template RNA or cDNA, selectively segregating nucleic acids with a different distinctive nucleic acid feature, thereby providing one or more further subpool(s) of nucleic acids,
• generating fragments of said segregated nucleic acid molecules by fragmenting or obtaining fragment copies of said segregated nucleic acid molecules, wherein the fragments of each subpool or combined subpools remain separable from fragments of other subpools or other combined subpools by physically separating the subpools or by attaching a label to the fragments of the subpools, with the label identifying a subpool, or determining a partial sequence of said segregated nucleic acid molecule and preferably aligning at least two sequences or partial sequences to a joined sequence.

## Description

The present invention relates to the field of analyzing complex mixtures of nucleic acids and sample preparation for characterization methods and sequencing, especially high throughput sequencing techniques, such as Next Generation Sequencing (NGS).

NGS is currently the foremost complete analyzing method. Next Generation Sequencing is a generic term for parallelized sequencing through polymerization as high-throughput DNA sequencing method. NGS reads sequences of up to many million fragments which are typically between 10 to several hundred base-pairs long. The complete sequence is obtained by alignment of those reads which is a challenging task. Some NGS methods rely on a consensus blue print held in genomic and/or transcriptomic databases. The quality of the results depends on length and number of reads, reading accuracy, quality of information in the reference database and applied bioinformatics algorithms. To date many reads provide just limited information. For instance many of the reads cannot be assigned uniquely and therefore are discarded.

In addition, the sequencing depth and therefore the detection of low abundant nucleic acids is limited. For the analysis of RNA this implies that in samples, which contain a multitude of different RNA molecules of different cells or cell populations or disease organisms, rare RNA or parts thereof are less likely to be retrieved. In fact, in transcriptomics rare RNA transcripts of even a simple organism are less likely to be detected and quantified.

In more detail, for generating detectable signals most NGS approaches must amplify individual RNA molecules or their DNA copy. Emulsion polymerase chain reaction (PCR) isolates individual DNA molecules using primer-coated beads in aqueous bubbles within an oil phase. Singularizing of DNA molecules, e.g. by rigorous dilution is another option. Another method for in vitro clonal amplification is bridge PCR, where fragments are amplified upon primers attached to a solid surface. Another option is to skip this amplification step, directly fixing DNA molecules to a surface. Such DNA molecules or above mentioned DNA coated beads are immobilized to a surface, and sequenced in parallel. Sequencing by synthesis, like the "old style" dye-termination electrophoretic sequencing, uses a DNA polymerase to determine the base sequence. Reversible terminator methods use reversible versions of dye-terminators, adding one nucleotide at a time, detecting fluorescence at each position, by repeated removal of the blocking group to allow polymerization of another nucleotide. Pyrosequencing also uses DNA polymerization, adding one nucleotide species at a time and detecting and quantifying the number of nucleotides added to a given location through the light emitted by the release of attached pyrophosphates. The sequencing by ligation method uses a DNA ligase to determine the target sequence. Used in the polony method and in the SOLiD^{®} technology, it employs a partition of all possible oligonucleotides of a fixed length, labeled according to the sequenced position. Oligonucleotides are annealed and ligated. The preferential ligation by DNA ligase for matching sequences results in a dinucleotide encoded colour space signal at that position.

NGS technologies are essentially based on random amplification of input DNA. This simplifies preparation but the sequencing remains undirected. The sheer complexity of the sample information - simultaneously obtained - is the key hindrance for unambiguous alignment of the reads. Therefore, complexity reduction is essential for increasing the quality of the results.

The classical route for DNA complexity reduction, e.g. employed during the human genome project, is to create BAC (bacterial artificial chromosome) clones prior to sequencing. Distinct stretches of genomic DNA are cloned into bacterial host cells, amplified, extracted and used as templates for Sanger sequencing. Production, maintenance and verification of large BAC libraries are laborious processes and associated with appreciable costs. Due to these impracticalities and the incompatibility with existing NGS platforms it is generally sought to avoid bacterial cloning.

Another option to reduce complexity is to first select polynucleic acids based on their respective sizes. Different approaches include, but are not limited to, agarose gel electrophoresis or size exclusion chromatography for fractionation. Small RNA sequencing approaches employ this method in order to obtain e.g. a fraction of RNA molecules called micro RNA (miRNA) sized between 15 and 30 nucleotides.

The probably most straightforward approach of complexity reduction is by limiting the amount of input nucleic sample to a single cell. Single-cell sequencing approaches rely on amplification reactions from highly dilute solutions, are incapable of actually reducing the complexity inherent to cellular content since it contains an entire transcriptome, and are based solely on a selection of the input cells.

A different method for reducing the amount of input nucleic acid to below the amount contained within a single cell sometimes is termed limited dilution. A genomic nucleic acid sample is first fragmented and then diluted to an extent where spatial distribution of the nucleic acid fragments within the sample volume becomes significant. Then subpools are created by taking such small volumes from the total sample volume that most sub-pools contain no nucleic acids, a few subpools contain one nucleic acid each and even less subpools contain two nucleic acids. This leads to singularization of nucleic acids and therefore to complexity reduction compared to the full length genome as each singularized nucleic acid is a fragment of a genome. Therefore an increased sequence assembly efficiency for the individual nucleic acid fragments containing sub-pools is gained. Assembly and scaffold building for large genomes thereby is facilitated. In transcriptome analysis such a limited dilution approach will not reduce the complexity introduced through variations in expression of different genes as each transcript molecule will occupy one subpool and therefore as many subpools are needed as molecules in the sample to display the entire transcriptome of a sample.

A further option is to sequence-specifically reject RNA, e.g. in a hybridization-based approach that removes ribosomal RNA from the entire RNA sample. As opposed to other fractionation methods that rely either on prior sequence information or are directed towards a certain RNA fraction (e.g. polyA selection), removal of rRNA does not bias the sequencing sample if e.g. mRNA is investigated. However, the mere removal of ribosomal RNA is restricted to RNA samples and cannot be scaled in terms of complexity reduction.

It is also possible to employ sequence-specific selection methods, e.g. by targeted sequencing of genomic regions such as particular exons. The idea behind such capture arrays is to insert a selection step prior to sequencing. Those arrays are programmed to capture only the genomic regions of interest and thus enabling users to utilize the full capacity of the NGS machines in the sequencing of the specific genomic regions of interest. Low density, on array capture hybridization is used for sequencing approaches. Such technology is not "hypothesis neutral", as specific sequence information is required for the selection process.

A similar positive selection can be used for targeted resequencing. E.g. biotinylated RNA strands of high specificity for their complementary genomic targets can be used to extract DNA fragments for subsequent amplification and sequence determination. This form of complexity reduction is necessarily based on available sequence information and therefore not hypothesis neutral.

Preparations of genomes that reduce the complexity of the sample have been disclosed in WO 2006/137734 and are based on AFLP technology (EP 0534858). AFLP has also been applied to double stranded cDNA that is derived from RNA. Here the double stranded cDNA is first cut by a restriction enzyme and then fragments are segregated. Even though the complexity of the nucleic acid fragments contained in each subpool decreases, in the majority of the cases each fragment of a nucleic acid will be segregated into at least two different subpools. This means e.g. that the subpool information cannot be used for assembly of the nucleic acids of the sample after sequencing, as likely each restriction fragment of a nucleic acid is in a different subpool. Therefore when restricting cDNA during cDNA AFLP information towards the full length of the cDNA gets lost. This ambiguity is further increased as for covering the sequence of most cDNAs with at least one restriction site a multitude of restriction enzymes must be used. In addition the transcriptome is only statistically covered in a cDNA AFLP approach as the pool of restriction enzymes may or may not cut a nucleic acid.

Sequencing of 16S rDNA or 16S rRNA sequences from mixed samples of microorganisms is in general employed for detection of rare species within these samples. By restricting the sequencing approach to a specific signature of microorganisms both complexity and information content are reduced. Frequently only phylogenetic information is obtained.

Tag-based identification of transcripts includes SAGE (Serial Analysis of Gene Expression) wherein sequence tags of defined length are extracted and sequenced. Since the initial creation of tag concatemers is a disadvantage for NGS, derived protocols are used omitting this step.

A related method is CAGE (Cap Analysis of Gene Expression). CAGE is intended to yield information on the 5' ends of transcripts and therefore on their respective transcription start sites. 5' cap carrying RNA molecules are selected before end-tags are extracted and sequenced.

Although only defined parts of the transcriptome are extracted for analysis SAGE and CAGE have their limitations because they do not allow for comprehensive segregation.

Therefore there is a need for methods that can provide for smaller fractions of a nucleic acid sample and provide for means to improve the sequencing procedure, in particular for improving detection of rare nucleic acid samples e.g. in pools of nucleic acids of high concentrations, which reduce the chance to obtain signals of rare nucleic acids.

Therefore the present invention provides a method of ordering nucleic acid molecule fragment sequences derived from a pool of potentially diverse RNA molecules comprising
- optionally reverse transcribing the RNA molecules to provide a pool of cDNA molecules,
- segregating nucleic acids from said template RNA or cDNA pool, selecting for potentially different templates with a distinctive nucleic acid feature shared by the segregated templates, thereby providing at least a first subpool of nucleic acids,
- optionally once or more further segregating nucleic acids from said template RNA or cDNA, selectively segregating nucleic acids with a different distinctive nucleic acid feature, thereby providing one or more further subpool(s) of nucleic acids,
- generating fragments of said segregated nucleic acid molecules by fragmenting or obtaining fragment copies of said segregated nucleic acid molecules, wherein the fragments of each subpool or combined subpools remain separable from fragments of other subpools or other combined subpools by physically separation from the subpools or by attaching a label to the fragments of the subpools, with the label identifying a subpool, or determining a partial sequence of said segregated nucleic acid molecules and preferably aligning at least two sequences or partial sequences to a joined sequence.

The inventive segregation step has the advantage that sub-pools of nucleic acids are provided and this subpool information can be used to improve further sequencing reactions, e.g. Next Generation Sequencing which is based on obtaining reads of small fragments of the nucleic acids or other nucleic acid characterization methods. It is possible with the inventive method that the subpool information can accompany the nucleic acids and the fragments and this information is used for alignment of the sequencing reads and the determination of the concentration of an individual nucleic acid sequence within the subpool. Furthermore, subpooling can reduce complexity to such a degree that transcripts of an organism and/or transcripts of different cells or cell populations and/or transcripts of different organisms that are present in a sample in different concentrations can be segregated in order to increase the likelihood of detecting rare nucleic acids within the sample of abundant RNA entities.

For unambiguous alignment of sequencing reads and the subsequent precise sequence assembly, efficient procedures for the reduction of sample complexity are required. A high degree of the complexity of the original material results from its disorder, the blend of sequences of different concentrations. Some advantages the inventive methods can provide are segregation methods which can
i) provide defined sub-pools of nucleic acid samples with common characteristics,
ii) provide means for coupling the sub-pool specific information to the nucleic acids and fragments thereof, and
iii) facilitate the concentration measurement of individual sequences within the sub-pool and consequently within the original sample,
to improve the quality of sequencing reads alignment and/or to analyze the original sample by other means.

With this method it is possible to reduce the complexity of transcriptomic samples to such a degree that rare transcripts can be detected within the main competing signal of all other, possibly highly abundant transcripts. The method is suitable to measure quantitatively sequences and fragments thereof from the very rare to the highly abundant forms.

The core of this invention is the sorting of a nucleic acid pool into sub-pools prior the fragmentation step (e.g. required by NGS), where all nucleic acids fragments acquire the additional sub-pool information of their parental molecule. This information can be maintained throughout the sequence reading, e.g. partial sequence determination. Then, every read contains the sequence and the sub-pool information, which provides main advantages during the read alignment procedures. One has to solve parallel just several smaller instead of one large "puzzle". The complexity of the task becomes significantly reduced. As a result, i) multi-position assignments are more unlikely, ii) the origin of more reads can be determined which have been formerly classified with "no-match", iii) in the case of transcript analysis, splice junctions and transcription start site variation are detected with higher probability, and iv) more full-length transcripts can be detected.

The sub-pooling of transcripts pools can be achieved through sub-pools with different additional information content. The gained benefits depend on the chosen methods.

Segregation into subpools can be performed by exploiting transcript properties as distinctive nucleic acid feature which are directly or indirectly sequence related. Such properties are for example the affinity to adsorbing matters like various column materials (e.g. silica gel) or the solubility in the presence of salts, polymers or other additives. In such indirect sequence related segregation the required information on the sample nucleic acids is limited, e.g. precipitation depends predominantly on length, the GC-content and secondary structures. The distinctive nucleic acid feature can be an adsorption or solubility property.

Alternatively or in addition, sub-pools can be generated through methods which utilize distinctive sequence information like i) partial internal or terminal sequences or/and ii) transcript size.
i) Using distinctive sequences (usually small nucleotide sequence portions) is the most powerful segregation tool. E.g. a distictive nucleic acid feature can be a partial sequence of the nucleic acids stemming from the template RNA or cDNA. The distinctive sequence can be a single nucleotide type (e.g selected from A, T, U, G or C) or more at a specific position within the nucleic acids to be segregated. E.g. nucleotides can be segregated for the presence of one or more nucleotide types or sequences at either the 5' or 3' terminus or in a given distance from said terminus. On one hand an array of hybridization probes, which covers one or more sequence possibilities of said distinctive portion of the nucleic acid, can be used to create sub-pools. Even if sub-pools contain different nucleic acids and some nucleic acids will be present in several sub-pools, such segregation approach already reduces the complexity of the original pool. After collecting all reads it is known to the aligment algorithm that the transcripts contain the subpool specific sequence(s), preferably the alignment algorithm must ensure, that all transcripts display at least one sub-pool specific sequence.

Segregation by selecting for a distinctive nucleic acid feature like a distinctive sequence (e.g. a single nucleotide or partial sequence at a specific position as described above) can be performed by either selecting such nucleic acids with the distinctive sequence or by specifically amplifying nucleic acids with said distinctive sequence and further utilising these amplicons in the inventive method.

A preferred segregation method uses the sequence information of both termini, thus start and end site of the nucleic acids. After termini-specific amplification and if the redundancy in the sequence specificity is zero (no mismatch allowed), then all sub-pools contain amplicons, e.g. PCR products, with exactly those termini. Hence, sub-pools can contain several nucleic acids of RNA molecules such as transcripts, but each nucleic acid is only presented in one sub-pool. By this means, the complexity of the alignment procedure is largely reduced.
ii) The RNA molecule size can be exploited to segregate the RNA according to the number of nucleotides per RNA via electrophoresis techniques (gel or capillary electrophoresis), or other methods. The later alignment of the different reads per sub-pool can benefit from the boundary condition of a certain rather narrow size range.

As used herein nucleic acid molecule derived from an RNA molecule refers to a nucleic acid of any type with the same sequence as the RNA from the sample.

In particular preferred during the segregation step full length or complete nucleic acids are segregated or selected from the template RNA or cDNA pool. Segregation of full length or complete nucleic acids at this step, prior to fragmenting, has the benefit that each segregated pool contains the sequence information of entire nucleic acids - even after fragmenting - which improves assembly of the sequence after sequence determination. "Full length" or "complete" in this context reads on the complete nucleic acids that are to be sequenced, e.g. as obtained after reverse transcription. It may comprise sequences of RNA starting from the 5' cap end up to the, but in most cases excluding the poly A tail, but may also relate to nucleic acids that are incompletely (reverse) transcribed, however without being artificially cut, e.g. by using endonucleases.

It is within the scope of this invention that the RNA was degraded or fragmented or digested by nuclease activity and the cDNA molecules derived from such RNA is only a partial sequence. Also the cDNA can be a partial copy of the RNA, e.g. oligo dT primed reverse transcription of mRNA is stopped before a full length cDNA copy is polymerized. This can be achieved through e.g. time restriction or through conditions where the reverse transcriptase stops polymerization at regions of secondary structure. Such a fragment can then be segregated by a common feature, e.g. the sequence preceding the poly A tail of an mRNA.

It is preferred that the pool of cDNA (cDNA libraries) contains nucleotides of the transcription start and/or end site, e.g. the first 25 and/or last 25 nucleotides. The pool of cDNA may also only consist of such first and/or end nucleotides. For example in CAGE (Shiraki-2003) 20 nucleotide tags are created that represent the 5' end of mRNAs. Of course such an approach will preclude the assembly of full length transcripts or the determination of their concentration. However such tags can be used to determine expression on a whole gene level, meaning the concentration of all transcription start sites can be measured. As only a short portion of an RNA will be sequenced sequencing depth increases and low level expressed genes will be more likely represented in the reads. However highly abundant transcripts will still be more often sequenced than low abundant transcripts. Therefore a segregation approach will increase the likelihood of low abundant start sites to be detected. For instance the short 5'tag sequences that are used to prepare a CAGE library can be segregated into fields of a matrix according to nucleotides on the 5' and/or 3' ends of such tag sequences. Therefore 5' tag sequences of low abundant transcripts will be more likely represented in a CAGE library that was prepared including a segregation step. Segregation can thus be performed on RNA, a cDNA thereof or other nucleic acids, e.g. RNA fragments, cDNA fragments or amplified nucleic acids therefrom.

The segregation step can be optionally repeated to obtain a different subpool with a different characterizing nucleic acid feature. This generation of further subpools can be performed sequentially or parallel to the generation of the first or other subpools.

The present invention in essence is a combination of selecting a pool of diverse RNA molecules, optionally generating cDNA, segregating the RNA or the cDNA, or any other nucleic acid derived therefrom, e.g. after amplification, optionally repeating the segregation for different parameters and fragmenting these segregated nucleic acids obtaining a pool of fragments. A fragment is considered a nucleic acid portion of shorter length than the complete nucleic acid molecule from which it is derived. Such fragments can be e.g. forwarded to Next Generation Sequencing approaches or other nucleic acid characterization methods. NGS is currently the foremost complete analysing method. However, the present invention is neither limited nor dependent on NGS. Other sequencing technologies can similarly benefit from the inventive segregation method.

Often not solely the complete sequencing of the nucleic acids is required to clearly characterize a certain sub-pool distribution. Any other methods like specific interaction with molecular probes or melting behavior can be applied to describe the original nucleic acid pool through a unique signature.

The invention improves the alignment of large numbers of individual sequencing reads to determine the sequence of nucleic acids and/or their copy number.

In one embodiment the generation of fragment (partial) sequences is done during the sequencing step, rather than first fragmenting and then sequencing such fragments. Here a random (universal) primer is used to prime the sequencing reaction within a single molecule. Therefore the sequencing reaction will in most cases create a fragment sequence from within the molecule. If the molecule had a subpool specific label this label could be read out after the sequencing reaction, providing the fragment sequence with the subpool specific label. The same molecule could be subjected to further sequencing, thus providing a multitude of fragment sequences that can be assembled to a contig or a full length sequence of the nucleic acid molecule, the RNA or transcribed cDNA. As a specific nucleic acid can be present in multiple copies, such sequencing could be done also in parallel. Here a multitude of random (or universal) primers prime the sequencing reaction of a multitude of nucleic acid molecules producing a multitude of fragment sequences that as a whole can be used to align or assemble the sequence of the segregated nucleic acids.

It is within the scope of this invention that fragments are ligated to each other prior to sequencing.

Nucleic acids are linear polymers of single nucleotides. These molecules carry genetic information (see triplet code) or form structures which fulfill other functions in the cell (e.g. regulation). The nucleic acids which are analyzed by the present invention are ribonucleic acid (RNA). RNA (sequencing) analytics is a particular difficult task due to the complexity of RNA populations in single cells. The invention relates to the identification (particular sequence determination) of all types of RNA in a cell, including mRNA (transcripts), microRNA, ribosomal RNA, siRNA, snoRNA.

The transcriptome is the set of all RNA molecules, or "transcripts", produced in cells. Unlike the genome, which is roughly fixed for a given cell line, the transcriptome varies with the kind of cell, tissue, organ and the stage of development. It can alter with external environmental conditions. Because it includes all transcripts in the cell, the transcriptome reflects the genes that are being actively expressed at any given time, and it includes degradation phenomena such as transcriptional attenuation. Transcriptomics is the study of transcripts, also referred to as expression profiling. An inventive benefit in using the inventive segregation method on RNA samples is that transcripts with low copy numbers or any other type of RNA which is present in the sample in a low concentration has an increased chance to be sequenced and analyzed in the subpool. One drawback of Next Generation Sequencing is that highly abundant nucleic acids reduce the chance that fragments of low concentration are sequenced. The inventive segregation allows the differentiation of high copy number entities with low copy nucleic acids. Thus preventing that such low copy nucleic acids are excluded from detection - or in any other preceding step such as e.g. during amplification.

The general principle is to reduce the complexity of a pool of nucleic acids by sequencing smaller segregated portions. These smaller portions are called sub-pools. In a preferred embodiment all sub-pools together contain all nucleic acids to be analyzed of the original pool. However, it is in principle not necessary to analyze all RNA molecules and thus some sub-pools can be ignored or are not even created/may remain empty. There are three main factors that contribute to the complexity of nucleic acids pools.

The first factor is determined by the combined length of the individual different sequences. Because the sequence is encoded through 4 bases (T and U are considered equivalent for carrying the same information) the complexity increases as a variation, equal to four to the power of length. However genomes contain redundant information like repeats or any other kind of order, e.g. that arises through the evolution of genes. Therefore different genes can contain stretches of the same or very similar sequences. This creates ambiguity in the de novo assembly of contigs or full length transcript sequences and limits the length of contigs that can be built. Even in alignment processes where a reference sequence is available such ambiguity restricts the alignment of individual reads. This ambiguity increases with decreasing read length of the sequencing process.

The second factor is determined by the number of different sequences within a sample. The complexity increases with the number of permutations, therefore with the factorial of different sequences. Two sequences have two possibilities to arrange, three sequences have six possibilities and so forth.

The third factor is the difference in copy numbers (transcript concentrations) and to lesser degree the amount of precognition about these differences, e.g. if it is known that the difference of certain copies is in the order of 1/1.000. Each different sequence belongs to a group which is characterized of having one particular copy number. The level of distribution of these groups determines the complexity which is introduced through concentration differences.

The inventive segregation can help to distinguish different RNA molecules of the original sample pool. This segregation step can also be repeated once or more. Repetition herein shall not be interpreted that additional segregation steps have to be performed after the first segregation step - which is of course one option - but also relates to performing one or more segregation steps simultaneously. Thus, one or more subpools are generated and in each subpool specific nucleic acids are present (or enriched) which share a common feature and all other nucleic acids without that shared distinctive nucleic acid feature can be excluded from each pool (or at least are not enriched).

These factors contribute directly to the difficulty of determining the correct sequence and concentration of all and in particular rare molecules within a sample. The general principal of the present invention is the constituting of sub-pools where these factors can be controlled, and simultaneously the complexity of the pool reduced, before sequencing reads are generated. Thus, the method simplifies the in-line sequence alignment. Sub-pools emerge through segregation methods which are within the scope of this invention.

In a preferred embodiment of the present invention the method further comprises determining the sequence or a partial sequence of the fragments of the first subpool and optionally further subpools. This sequence of the fragments or a portion thereof can be determined by any suitable method known in the art. Preferred are sequence determination methods that can be scaled to high throughput sequencing methods, in particular Next Generation Sequencing. In such a method sequence length of at least 5, preferably at least 8, at least, 10, at least 15, at least 18, at least 20, at least 22 nucleotides of the fragments or more can be determined. Preferably the full length sequence of the fragments are determined. If only portions of the fragments are sequenced this can be either portions of the 5' or the 3' end or internal portions which can be selected for with specific or unspecific (e.g. random) primers.

Determining a partial sequence of a nucleic acid preferably comprises determining a sequence portion of at least 10, preferably at least 15, at least 18, in particular preferred at least 20, even more preferred at least 25, nucleotides but excludes determining the complete sequence of the nucleic acid. According to the present invention it is possible to either generate fragments of the segregated nucleic acid molecules by fragmenting or obtaining fragment copies (e.g. amplifying portions of the nucleic acid molecules) and subsequently determine the sequences thereof or to determine a sequence or partial sequence of a fragment of said segregated nucleic acid molecule and, preferably align at least 2, preferably at least 3, in particular preferred at least 4, at least 6 or at least 8 sequences or partial sequences to a joined sequence. According to this option, it is not necessary to physically provide such fragments but possible to only obtain sequence portions which can be determined from the nucleic acid molecules themselves without a physical fragmenting step and create a joined sequence by aligning such partial sequences. According to this embodiment, it is thus not necessary to provide specific labels providing the information of the segregated pool since the sequences are determined directly on the nucleic acid molecules of the sub-pool. This is possible by e.g. random priming, a primer extension from inside the nucleic acid molecule, or by e.g. nano-pores which can read out at any point of the sequence, therefore creating "fragment reads". Such reads can then be aligned as described herein.

In particular, it is not always necessary to provide full-length sequences of all fragments provided. It is also possible to determine missing sequence portions from other fragments which may e.g. overlap and thus provide the same sequence as is lacking in the incompletely sequenced fragment. E.g. it is usually more efficient only to determine the sequence from one end of the fragment and to sequence a partial sequence as mentioned above of e.g. at least 10 nucleotides. Such partial sequences can then be aligned to a joined sequence. Although according to one embodiment it is possible to determine the full-length sequence of the segregated nucleic acid molecule by the method of the present invention, it is also possible to only determine portions thereof long enough to identify said nucleid acid molecule.

Preferably the information about the sub-pool origin escorts the nucleic acid molecule and each of its fragments during the sequencing run. On one hand the sub-pool information can be passed on through labeling. Every fragment may receive an identifying nucleotide sequence (e.g. adding a subpool specific sequence tag of e.g. 1, 2, 3, 4, 5, 6, 7, 8 or more subpool related nucleotides), reporter module like a fluorescent dye, nano-dots, or others. It is preferred that the subpool specific label is a nucleotide sequence (barcode) that is added to the fragment. Furthermore it is preferred that the barcode is read out after or during sequencing the nucleic acid fragment. On the other hand, the sub-pool information can be perpetuated through spatial or temporal separation, which means that each sub-pool is sequenced in a different area (cluster on a slide) in the machine or discriminating time slot, e.g. each subpool may be sequenced sequentially. No additional process conduct is needed for most of those procedures. In the case of individual labeling with a reporter molecule, the reporter signal has to be identified and connected to the read.

The individual sub-pools can be sequenced separately. Reads of each sub-pool are aligned either to the genomic blue print, or they are aligned de novo by comparing them with all other reads within the same sub-pool and not the total pool. Therefore, the complexity of the original sample pool is greatly reduced.

Abundant RNA molecules, in particular transcripts, interfere only in one sub-pool of their appearance and so compromise its reading depth, but not the rest of the subpools. Because the probability of reading individual fragments is proportional to their relative concentration within the pool or sub-pool respectively, fragments which are present in just a thousandth will on average only be read once while having read the other fragment(s) thousand times.

For alignment of the reads, all reads are grouped, and where possible oriented according to their sub-pool address. Second, all reads are aligned to each other or to the blue-print sequence data base. The alignment must fulfill all boundary conditions, if e.g. further information of the complete sequence such as length is known in addition to the sub-pool information.

However, often it is not necessary to completely sequence a fragment but only obtain a portion of its sequence. Sometimes such a portion is sufficient to identify the nucleotide or align other sequenced portion of other fragments to a full sequence (e.g. if the fragments contain overlapping sequences).

Apart from sequencing portions of the fragments it is also possible to only obtain fragments i.e. smaller nucleic acid molecules with portions of the original nucleic acid, and determine the sequence or a portion therefrom. "Generating fragments of said segmented nucleic acid molecules" thus, also relates to obtaining a fragment which contains any kind of sequence portion. Fragmenting can be by e.g. physical means be either in a sequence dependent way, e.g by endonuclease digestion or by sequence independent means such as by a physical means like sonication or shearing. Generating the fragments further relates to obtaining fragment copies. The nucleic acid molecule can be e.g. amplified to further copies which are in turn fragmented. If a random fragmenting process is used this can result in generation of different fragments for each nucleic acid molecule. On the other hand, if a sequence dependent method is used, e.g. restriction endonuclease digestion or sequence specific amplification all fragments of one nucleic acid molecule will be the same. Furthermore, it is possible to generate fragments by amplification, i.e. sequencing fragments. This can e.g. also be done in a sequence independent or dependent method, in particular preferable by random priming in order to obtain internal sequence portions with said fragments. Example sizes of the fragments or determined partial sequences are e.g. at least 10, at least 20, at least 25, at least 30, at least 35, at least 40 nucleotides. Fragments or determined partial sequences can be up to 20,000, up to 10,000, up to 5,000, up to 4,000, up to 3,000, up to 2,000, up to 1,000, up to 800, up to 700, up to 600, up to 500, or up to 400 nucleotides long. The preferred ranges are of 10 to 10,000 nucleotides, preferably of 25 to 500 nucleotides.

It is within the scope of this invention that fragments are joined prior to sequencing. It is preferred that such joined fragments are interspersed by different sequence stretches that allow sequencing primers to prime consecutive rounds of sequencing.

The segregated nucleic acid molecule or the nucleic acid molecule to be segregated can be either single stranded or double stranded. In cases where single stranded molecules are segregated the strandedness of a fragment in relation to its parent molecule is clear as it has a 5' and a 3' end. When double stranded nucleic acid molecules are used then there needs to be a distinguishing property on one strand but not the other (e.g. methylation) as the double strand has a 5' and 3' end on both strands. In cases where a feature (preferably a sequence portion) at the 5' and/or 3' end of the RNA or cDNA was used as the nucleic acid feature the orientation of the molecule is still known before fragmentation. Therefore one of the two strands can be used for fragmentation. One of the two strands can be selected for by any means known to the art. E.g. the end of one strand can be labeled during the segregation. For instance one of the PCR primers may contain a labeling group such as biotin and be selected for afterwards using column chromatography with an avidin coupled matrix. Another possibility is to use one primer that has a 5' phosphate and another primer that has no 5' phosphate and subject the PCR products to a lambda exonuclease that preferentially will digest the strand that has a 5' phosphate. By preserving the strandednesss or the strand information of the nucleic acid molecule throughout the segregation and fragmentation, the performance of subsequent assembly or alignment is improved. For instance if the strandednesss of the fragments is preserved, each fragment can be aligned to the plus or minus strand of the genome, thereby distinguishing between sense and antisense transcripts. The same holds true for cluster building or the de novo assembly of transcripts as again sense and antisense clusters/transcripts can be distinguished. It is therefore preferred that during fragmentation the strandedness or strand information is preserved, preferably by selecting for one strand, e.g. through a lambda nuclease digest of the other strand. It is possible during segregation to select one strand to be segregated (either the sense or anti-sense strand) or to label the selected strand in order to maintain strand information. Preferably the fragments of the selected strand are labeled according to the strand information and possibly also for pooling information (e.g. bar-coding as mentioned above).

In further preferred embodiments at least 2, 4, 5, 6, 7, 8, 9, 10, 12, 14, 16, 18, or at least 20 nucleotides, in particular consecutive nucleotides of these fragments are sequenced.

The original pool of potentially diverse RNA molecules can be of any source, in particular of any biological sample, preferably of a virus, prokaryote or eukaryote. The inventive complexity reduction method is important for any sorts of RNA sequencing approach, even when using a single cell which contains a diverse transcriptome but of course also samples which contain more than one cell, in particular samples of diverse origin, e.g. containing many different cells of diverse organisms or similar cells with different or modified gene expression (e.g. tumor cells).

In a particular preferred embodiment of the present invention the nucleic acid feature used for segregation is a given nucleotide type, preferably selected from any one of A, T, U, G, C, at a certain position in the nucleic acid molecule, preferably the position being within 100 nucleotides from either the 5' or 3' terminus of the nucleic acid molecule. Such methods, that select for one specific nucleotides, e.g. to obtain full length sequence source disclosed in the WO 2007/062445 (incorporated herein by reference). According to the present invention it is possible to amplify or select for specific nucleic acid molecules in a segregation step by using, e.g. a primer, which is specific for e.g. one end (either the 3' or 5' end) of the RNA or cDNA and containing one or more further nucleotides specificities which act to segregate the nucleic acid molecules according to the complementary nucleotides after the (universal or wobble) primer portion. If full length RNA should be segregated then it is possible to use primer specific for the ends, e.g. the polyA-tail (or polyT-tail on a cDNA corresponding thereto) or to attaching artificial tails onto the RNA or cDNA and using primers specific for this tail. The primers can be specific for the next 1 to 100, preferably 1 to 10 nucleotides, e.g. the next 1, 2, 3, 4, 5, 6, 7, 8, 9 or 10 nucleotides. By using wobble nucleotides on said primers it is also possible to select for specific nucleotides after these ends. Preferably the specific distinguishing nucleotides are within the first 100 nucleotides from either the 5' or 3' terminus of the nucleic acid molecule. It is of course also possible to use primers to select any internal region wherein the nucleic acid molecules can be separated in the segregation step.

The same principle mentioned above for primers of course also applies for oligonucleotide probes which can be specific for such a distinguishing nucleotide type.

Preferably, the nucleic acid molecules are selected for common nucleotides within the 10 nucleotides next to the 5' and/or 3' terminus, preferably for one or more common 5' and/or 3' terminal nucleotide types.

These primers or probes preferably are used in combination with primers or probes which are selected for a different nucleic acid feature. Such primers can e.g. be used separately or sequentially to generate subpools specific for the nucleic acid feature. Such primers or oligonucleotides used in a combination (i.e. "primer matrix") can e.g. be primers which have a universal part and a distinguishing part wherein the distinguishing part is e.g. A in the first primer, T in the second primer, G in the third primer and C in the fourth primer. Preferably, more than one nucleotide is used as the nucleic acid feature and the combination can e.g. be primers or oligonucleotide probes ending with AA, AT, AG, AC, TA, TT, TG, TC, GA, GT, GG, GC, CA, CT, CG, or CC, thus separating nucleic acids with complementary nucleotides into different sub-pools. In a further preferred embodiment the nucleic acid feature contains 3 or more, e.g. 4, 5, 6, 7, 8, or more specific nucleotide types. In a further preferred embodiment, combinations of primers are oligonucleotides selecting for distinguishing nucleotides at both the 5' and/or 3' terminus, e.g. both primers or probes being specific for the two or more 5' nucleotides and the two or more 3' nucleotides.

As mentioned above it is also possible to select for internal regions wherein it is also possible to use a combination of such a primer pair which selects for two nucleotide types on each side of the amplicon. Internal regions can alternatively also be selected for by using end-specific primers or probes having a certain number of unspecific nucleotides (e.g. wobble or universal nucleotides) prior to the complementary nucleotides for the specific internal region.

In a preferred embodiment the nucleic acid feature that is used for segregation, is used during the assembly (or alignment) of short reads as a qualifying property of the assembled (or aligned) sequence. For instance if the nucleic acid feature was a certain length or length range then the qualifier for a correctly assembled sequence would be such length or length range. If the nucleic acid feature was a certain sequence then when sequencing fragments of this nucleic acid, that are e.g. 36 bases long, then in addition to the 36 bases another n bases are known for each fragment, where n is the number of bases of the nucleic acid feature. If for instance the nucleic acid feature was 6 known bases on the 5' side and 6 bases on the 3' side of the molecule then in addition to the 36 bases of each fragment 2x6 bases are known to be within a certain distance (the length of the fragmented molecule) from the sequenced fragment. Therefore if the nucleic acid feature was a certain sequence then this sequence must be again contained within the assembled sequence. It is preferred that the nucleic acid feature is at a certain position of the segregated nucleic acids, preferably at a certain distance from the 5' or 3' end of the template RNA or cDNA. Preferably the nucleic acid feature is a sequence and the sequence is used during assembly. The nucleic acid feature may comprise two sequence portions, e.g. of 2, 3, 4, 5, 6, 7, 8, 9, or 10 known nucleotides, positioned in a certain base distance, e.g. in a distance of e.g. 20 to 10000 nts, preferably 30 to 5000 nts, in particular preferred 50 to 1000 nts.

In a preferred embodiment the segregated nucleic acids contain the full length sequence of the template RNA or cDNA. This will greatly increase the de novo assembly of contigs or even full length sequences as all fragment reads generated during a sequencing process can be aligned within a subpool, i.e. with the fragment or partial sequences obtained from one subpool.

If the nucleotides of the 5' and/or 3' end of the template full length RNA were used as the nucleic acid feature(s)for segregation, the nucleotides of the start and/or end site of the full length RNA molecule are known for all fragments of such subpool. Such information allows e.g. to position fragments or their assembled contigs correctly on the plus or minus strand of the genomic DNA, thus separating sense and antisense transcripts of a gene. In preferred embodiment the RNA molecule used according to the inventive method is a full length RNA. Full length RNA can e.g. be selected with the above mentioned method. The same also applies to full length cDNA corresponding to the full length RNA. As used herein the term "full length RNA" or "full length cDNA" is defined as RNA or DNA that includes a sequence complementary to the RNA sequence from the first base to the last base of the RNA. Such a method is e.g. disclosed in WO 2007/062445 (incorporated by reference) and comprises amplification selective for end specific nucleic acid features e.g. by performing a segregated amplification or selection (as described herein) on full length RNA. In case of RNA molecules that have a cap and/or a tail (polyA-tail) as is the case for most eukaryotic mRNA, "full length RNA" is defined as RNA that includes a sequence complementary to the RNA sequence for the first base after the cap e.g. the RNA 7-methylguanusin cap to the last base before the tail, polyA-tail, of the RNA template.

In order to bind primers during amplification and/or sequencing reactions to ends of nucleic acids or the fragments it is possible to attach linkers or adaptors to the nucleic acid molecules or fragments to allow primer binding.

By ordering a pool of RNA molecules into the inventive sub-pools it is possible to highly decrease complexity of the original sample, generating subpools with fewer nucleic acid entities and therefore increase the chance of successful sequencing and assembling afterwards.

In preferred embodiments the nucleic acids are divided into subpools wherein at least 10% of all subpools comprise the average amount of nucleic acids of all subpools +/- 50%. By employing a suitable segregation method for the given sample to divide the nucleic acids evenly into the subpools the complexity reduction method is sufficiently used. Of course, further subpools may exist wherein fewer nucleic acids are present, e.g. even empty subpools without any nucleic acids of the original pool which can be used as control reference. In preferred embodiments at least 15%, at least 20%, at least 25%, at least 30%, at least 35%, at least 40% of all subpools comprise the average amount of nucleic acids of all subpools +/- 50%. This error margin of +/-50% is in preferred embodiment up to +/- 50%, up to +/- 45%, up to +/- 40%, up to +/- 35%, up to +/- 30%, up to +/- 25%, up to +/- 20%.

Preferably the sample comprises at least one, preferably two, 3, 4, 5, 6, 7 or 8 rare RNA molecules. Rare can mean a concentration of below 1%, below 0.5%, below 0.1%, below 0.05%, below 0.01% (100ppm), preferably below 50ppm, below 10ppm, below 5ppm, below 1ppm, below 500ppb, below 100ppb or below 50ppb. Preferably at least 1, preferably 2, at least 4, at least 6 or at least 8 rare nucleic acids are in the sample to be analyzed.

In a further embodiment the nucleic acids are divided in subpools wherein at least 10% of subpools contain 2 or less nucleic acids, preferably 1 nucleic acid. Such a high dilution is in particular favorable for very rare nucleic acids that would be hard to detect if further nucleic acids would be present from the original pool, in particular in the original concentration.

In a further preferred embodiment the step of segregating the nucleic acids comprises specifically amplifying the nucleic acids from said template pool. In particular, the amplification is performed by nucleotide extension from a primer, preferably by PCR, in particular preferred wherein the amplification is performed by nucleotide extension from a primer, preferably by PCR, in particular preferred wherein the amplification is performed by using primers which select for at least one, preferably at least two, in particular at least two adjacent, different nucleotides after an unspecific primer portion whereby nucleic acid molecules are amplified which comprise the selected nucleotide as the nucleic acid feature specific for a subpool.

The above mentioned fragmentation step of the inventive method may be the first step used for sequence determination steps. Determining the sequence of the nucleic acids of the sub-pool may comprise, fragmenting the nucleotide molecules of the subpool as mentioned above, attaching a subpool specific label to each fragment of a given subpool, determining nucleotide sequences of fragmented polynucleotides of combined pools (or alternatively determining nucleotide sequences of separate pools with or without attaching a label), assigning fragment sequences to a nucleotide molecule depending on a subpool-specific label and overlapping sequences with other fragments, thereby determining the sequence of the nucleic acids.

Thus, in preferred embodiment subpool-specific labels are attached to the fragments. The subpool-specific labels can be nucleotides, which are preferably co-determined during sequence determination.

In further preferred embodiments the nucleic acids of the original pool are divided into at least 2, preferably at least 3, at least 4, at least 5, at least 6, at least 7, at least 8 subpools during the segregation step, which nucleotides each share a different nucleotide characteristic for each subpool.

In preferred embodiments primers or probes used for selecting nucleic acids in the segregation step are preferably immobilized on a solid surface, in particular a microarray or chip. The same type of segregation as described above for the distinguishing the nucleic acids can also be performed for distinguishing different fragments during the sequencing step.

In a particular preferred embodiment the inventive method further comprises amplifying the nucleic acid molecules, preferably after segregation, prior to determining the sequence, in particular preferred wherein said amplification is by PCR and at least one nucletide molecule is amplified to the saturation phase of the PCR. In particular preferred at least 10% of the different nucleotide molecules are amplified to the saturation phase of the PCR. Such an amplification reaction can be used to normalise the concentration of nucleic acid molecules in the pool or sub-pool. A PCR reaction e.g. has an exponential phase in which the nucleic acid molecules are essentially doubled in each PCR cycle. After the nucleic acid molecules reach a certain concentration in relation to the primer concentration, competitive reactions start to inhibit the amplification. Thus, amplification of abundant nucleic acid molecules starts to slow down due to the self inhibition of the nucleic acid molecules which can prevent primer binding. This phase is called the saturation-phase.

Preferably, highly abundant nucleic acid molecules reach this saturation phase and are inhibited from amplification whereas low abundant molecules continue amplifying exponentially. Preferably at least 10%, in particular preferably at least 20% of the different nucleic acid molecules enter this saturation phase. These amplification reactions can e.g. be monitored by using qPCR (quantitative PCR). Of course, said reactions occur in normal PCR reactions (but may be unmonitored) or other amplification reactions with self inhibition, e.g. after 20, 22, 24, 26, 28, or 30 amplification cycle, which are preferred minimum cycle numbers for the inventive amplification.

The inventive sub-pooling procedure can also be used to remove high copy transcripts, e.g. exclude sub-pools with high abundant nucleic acid molecules from sequence determination. Preferably, such sub-pools with high abundant nucleic acid molecules that are excluded from sequence determination are sub-pools comprising more than 100%, particularly preferred more than 150%, even more preferred than 200%, particularly preferred more than 300%, e.g. more than 400%, such as more than 500%, particular preferred more than 1000%, nucleic acid molecules above the average amount of all sub-pools which may contain all the nucleic acid molecules of the sample. Such sub-pools can e.g. be subpools which comprise nucleic acid molecules that constitute e.g. more than 0,1%, more than 0,5%, or even more than 1%, e.g. more than 2% or more than 5% or more than 10% of the entire original pool. Abundant transcripts to be excluded or normalized by this way are e.g. of housekeeping genes, GAPDH, actin, tubulin, RPL1, ribosomal proteins, or PGK1.

The present invention is further illustrated by the following figures and examples without being limited thereto.

### Figures:

Fig. 1: Workflow of the Segregation-NGS method for RNA.
Fig. 2: Simulation of the number of genes as function of the mRNA's (total copy numbers of all gene transcripts) through a log-log-normal function. Active genes G, 16,657, total transcripts T, 3.8 Mio, most common transcript number, 10, scale value of the log-log-normal function µ, 1 and shape parameter δ, 0.4.
Fig. 3: Exponential decay function describing qualitatively the relationship of the number of transcripts vs. genes according with the parameters tₛₜₐᵣₜ, 33, t_{end}, 1, the sum of all genes, 25,200 and a 4-fold amount of transcripts (100,269).
Fig. 4: Exponential decay function describing dependency of the mRNA's (copy number) vs. transcripts according with the parmeters cstart, 10,000, c_{end}, 1, decay constant τ of 0.0522, the sum of all transcripts is 100,128 and the sum of all copy numbers is 3.8 Mio.

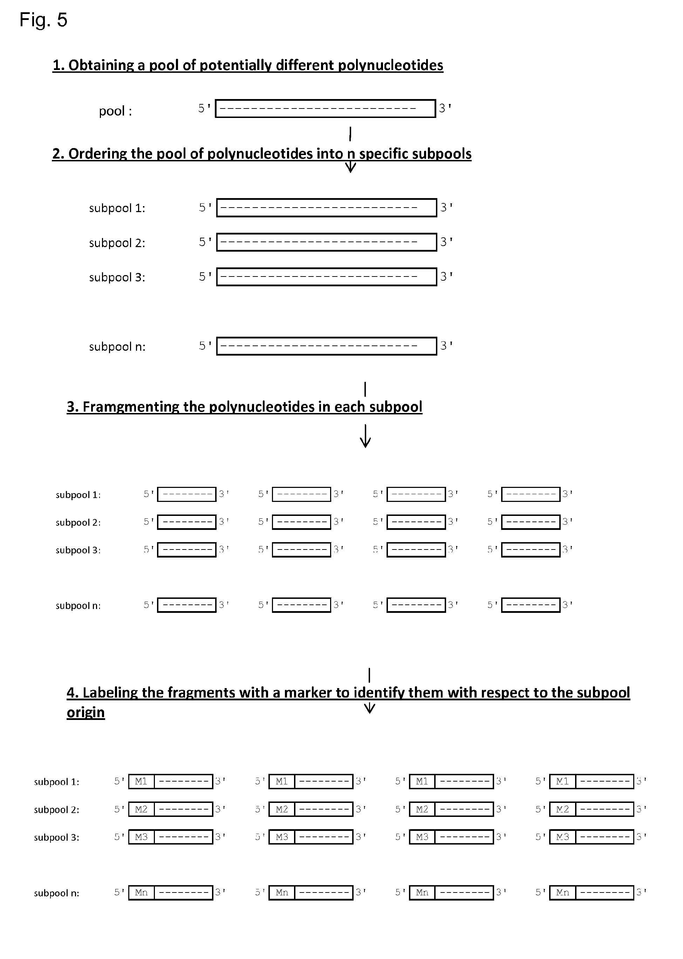

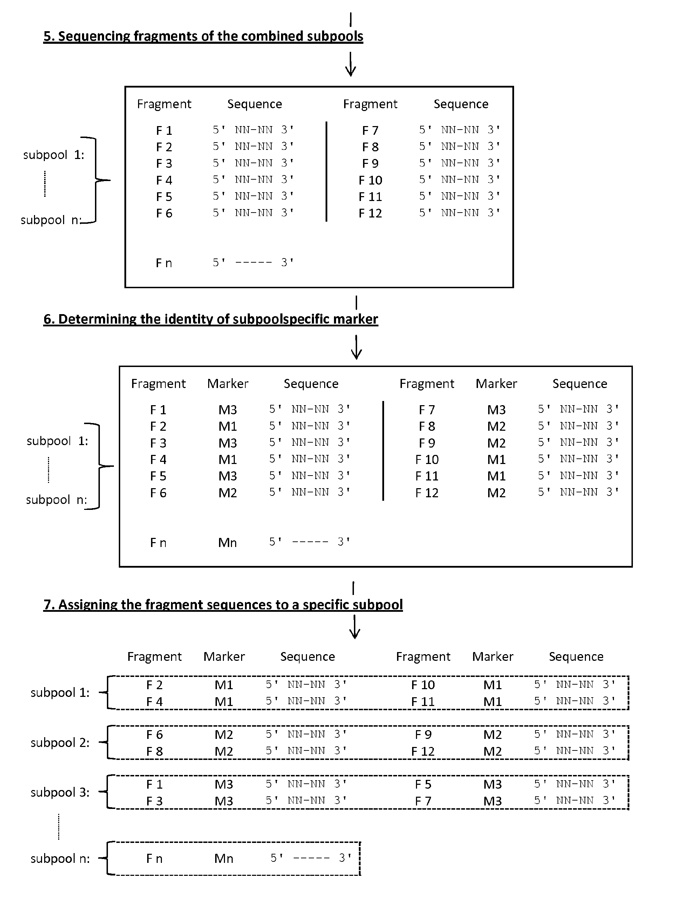

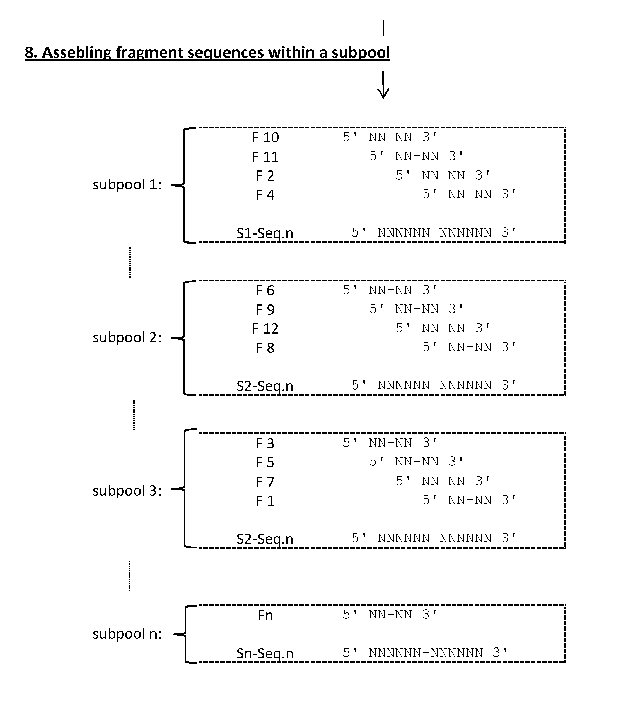

Fig. 5: General subpooling and fragmentation workflow.

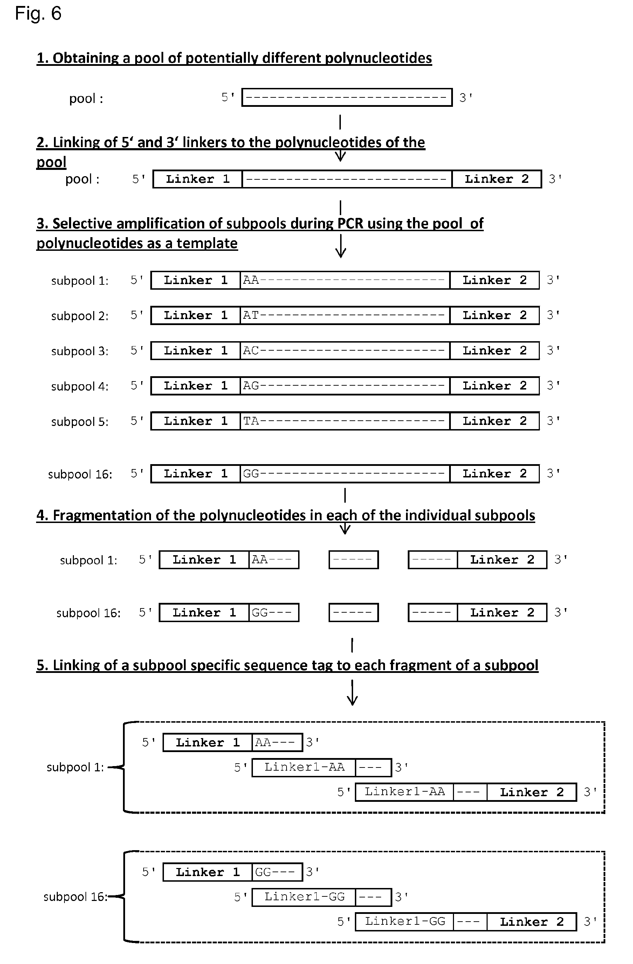

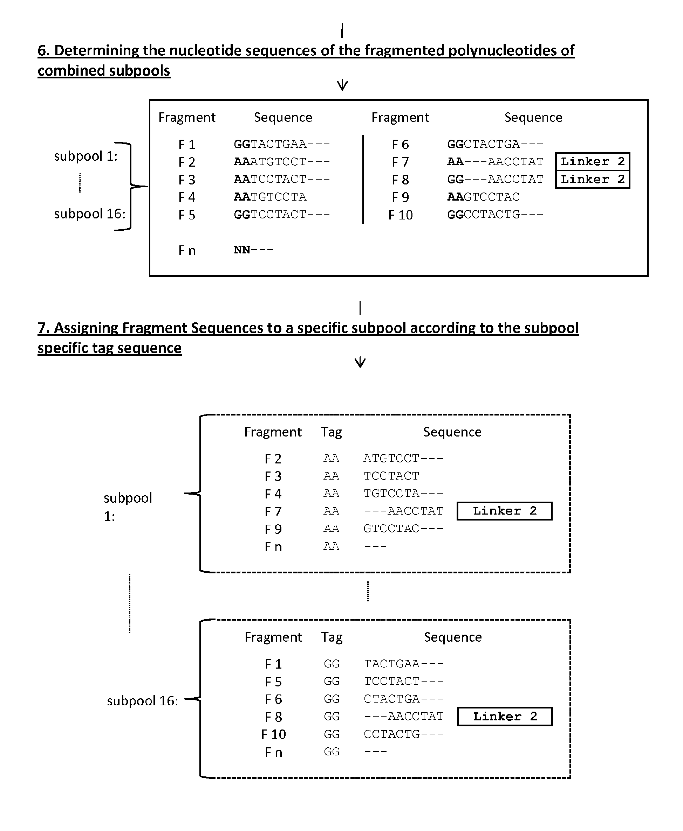

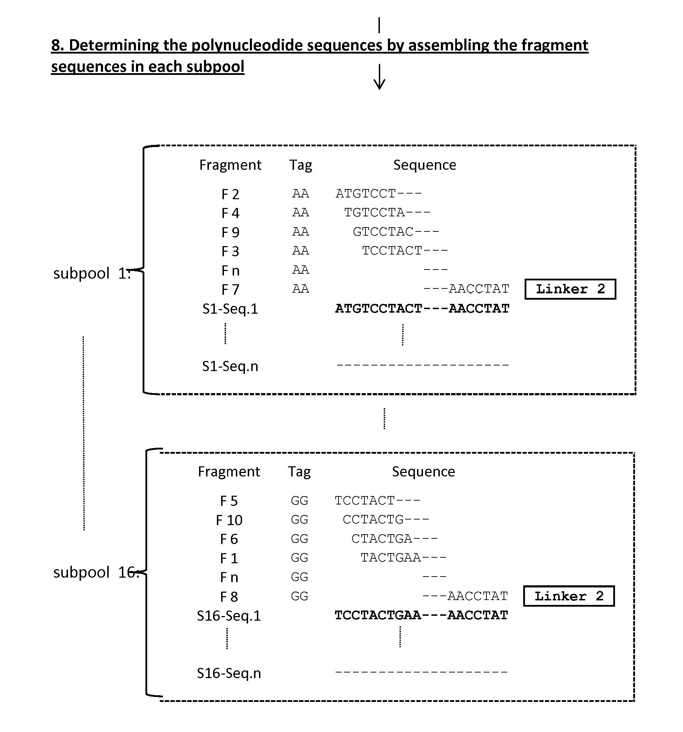

Fig. 6: General principle using nucleotide specific amplification (segregation). In this example the first two nucleotides at the 5' end used to define the subpools, also become the sequence tag.

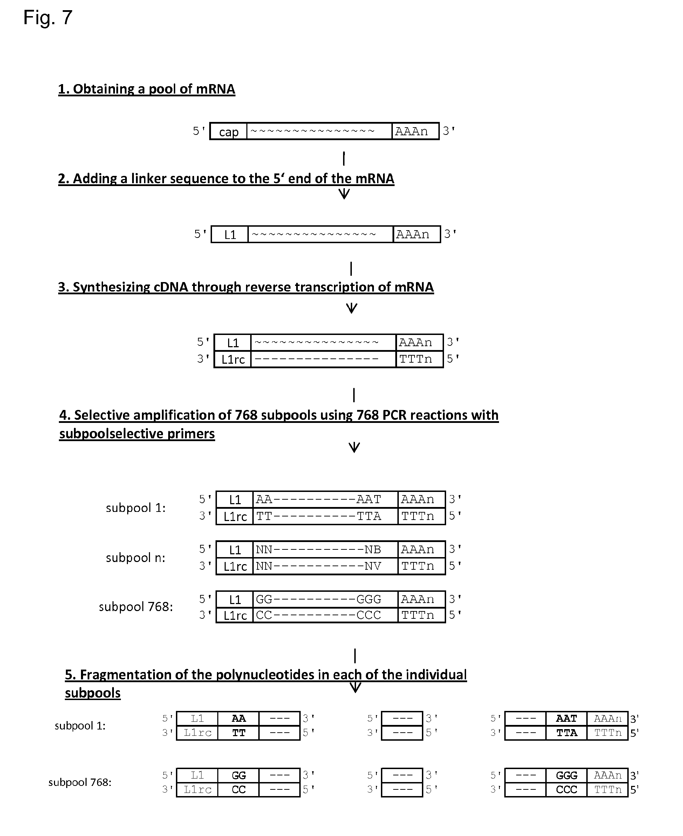

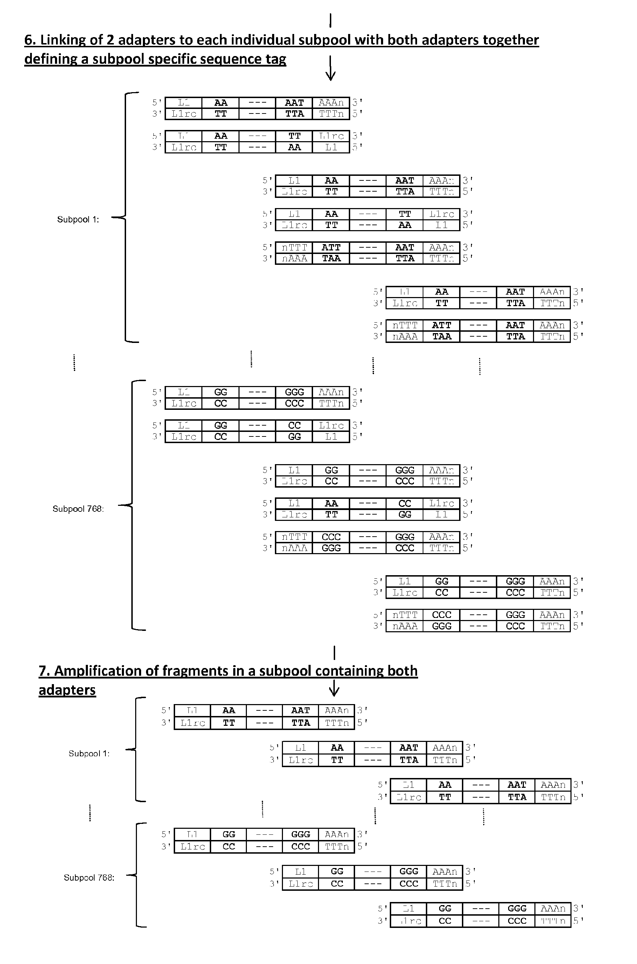

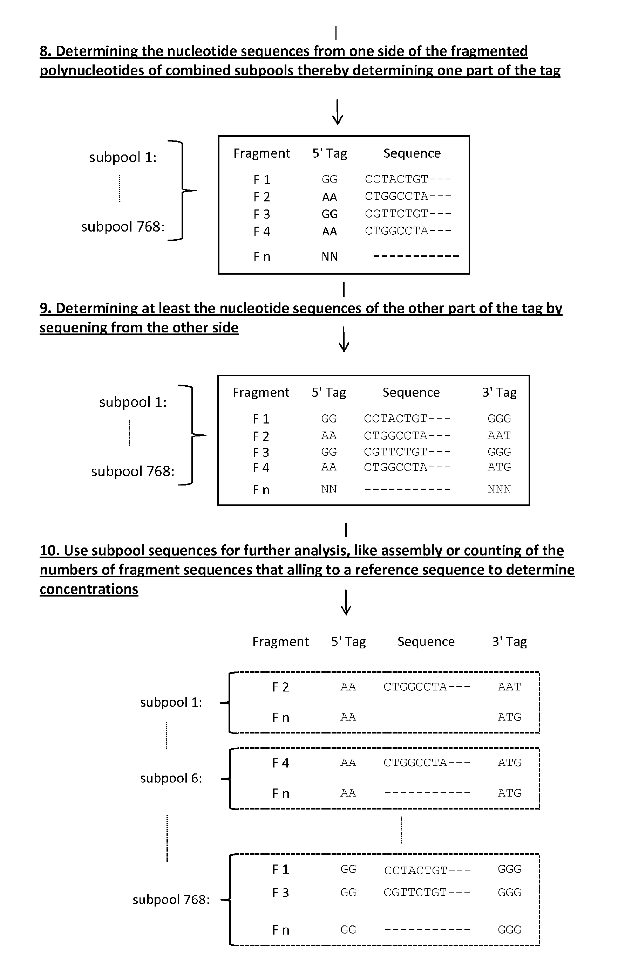

Fig. 7: RNA matrix segregation. In this example it is noteworthy that fragments F2 and F4 are sequence identical and could not be distinguished unless the segregation into the sub-pools was performed (see step 10). Adding a linker sequence to the 5' end of the mRNA as shown in step 2 can be done by any methods known in the art, such as Oligo capping (Maruyama 1994).

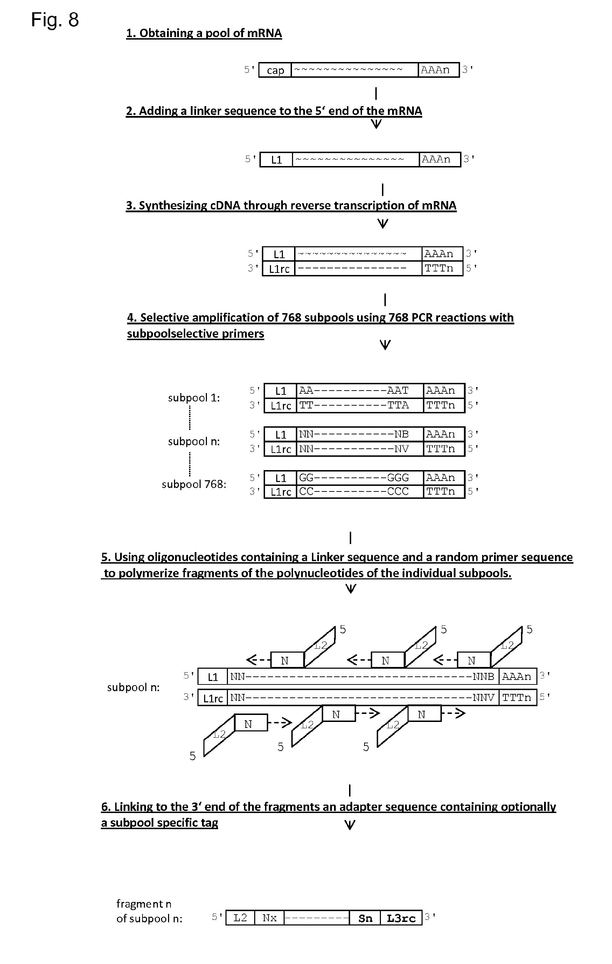

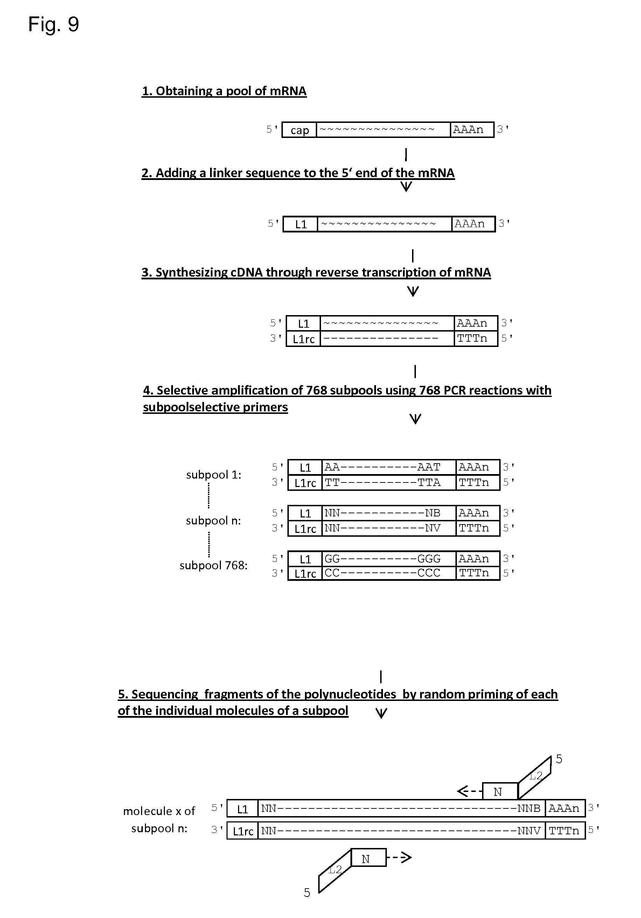

Fig. 8: Creating fragments by random primed polymerization Steps 1 to 4 are the same as in Fig 9. Shown is only subpool n. Sn in step 6 represents the subpool specific tag.
Fig. 9: Random primed sequencing, producing fragment reads. Steps 1 to 4 are the same as in Fig 7. In this example the molecule x of the subpool n is double stranded, each strand can serve as a template for sequencing. The random primer is bound to the surface of the sequencing chip. Single strands of each molecule of a subpool are hybridized to the primers on the chip. As the random primers can hybridize to any part of the molecule, the sequencing will produce "fragment" reads from the molecule.

### Examples

### Example 1: cDNA segregation by end-specific matrix separation followed by NGS analysis

2 µg of purified total RNA of a mouse (C57Bl/6) liver sample was primed by an oligo that contains a V (being either C,G or A) anchored oligo-dT sequence (Linker2-T₂₇-V) at its 3' end and reverse transcribed to generate cDNA. Employing the template-switch activity of the reverse transcriptase a linker sequence was added during reverse transcription reaction to the 3' end of the cDNA through reverse transcribing a 29 nucleotide long template switch oligo (Linker1) (US 5962271, US 5962372). Then the 5' end of the generated cDNA comprises a polyT-stretch introduced by the oligo which corresponds to the mRNA's original polyA-tail plus the Linker2 sequence. The 3' end of the cDNA comprises the reverse complement to the Linker1 sequence. Two different sets of samples were prepared for sequencing.

The single sample of comparative set A (without segregation) was prepared by PCR-amplifying in a 50µl reaction about 27 pg of cDNA to a level of about 800 ng using primers that hybridize to the template switch sequence (Linker1) at the 3' end and the polyT sequence at the 5' end of the cDNA (Linker2-T₂₇). To generate enough material for the subsequent sequencing sample preparation, eight purified PCR reactions were mixed and about 5 µg are further processed. In its essence, this sample contained a non-specific matrix with only one field therefore representing an amplification where the whole cDNA could have serve as a template.

Set B (with segregation) consists of 6 samples that correspond to 6 subpools of a 12 subpool matrix (4x3). To generate 12 subpools one of four primers with a 3'-terminal A, G, C or T specific for the 3' end of the cDNA and one of three primers with a 3'-terminal A, G or C specific for the 5' end of the cDNA was applied to selectively amplify in each matrix field only cDNA molecules with one specific termini combination. To generate the 6 samples of set B only 6 primer combinations were used (T₂₇-C / Linker1-G; T₂₇-G / Linker1-G; T₂₇-A / Linker1-A; T₂₇-C / Linker1-C; T₂₇-_{G} / Linker1-C; T₂₇-A / Linker1-T), each amplifying about 27pg of cDNA to 800 ng; 5 µg of 8 pooled replicates for each primer combination was used in subsequent reactions. In its essence, each of the 6 PCR samples of set B on average used a 1/12 of the cDNA as a template.

To prepare the samples of the two sets for next generation sequencing, each of the PCR sample was fragmented (by sonication) into fragments which are on average 200-1000 bp long. Afterwards, the samples were subjected to a standard Illumina genomic DNA sequencing sample preparation pipeline using an Illumina Genomic Prep Kit (#FC-102-1001; Illumina Inc., USA). In essence, adapters were added to the ends of the fragments, which are used to bind the samples to the flow cell, allow for cluster generation and enable the hybridization of a sequencing primer to start the sequencing run. In addition the 6 samples of set B were bar-coded with standard Illumina multiplex tags using the Multiplexing Sample Preparation Oligonucleotide Kit (#PE-400-2002; Illumina Inc., USA). Adapter ligated fragments in a size range of 200-600 bp were size selected for sequencing. The single sample of set A was loaded onto one channel of the flow cell and the 6 samples of set B mixed in equal amounts and loaded onto a second channel. Cluster generation was carried out on a cBot Instrument (Illumina Inc., USA) using the Cluster generation Kit(#GD-203-2001,version 2; Illumina Inc., USA). Then a 76 bp sequencing run was carried out on a GenomeAnalyzer II (Illumina Inc.) using the Sequencing Reagent Kit (#FC-104-3002, version 3; Illumina Inc., USA).

The multiplex tags of the 6 samples of set B were read out using Multiplex Sequencing Primer and PhiX Control Kit (#PE400-2002,version 2; Illumina Inc., USA).

For each of the channels, short (76 bp) reads were obtained, and the multiplexed reads of set B were separated according to their barcodes.

Then the number of reads for both data sets was normalized by randomly drawing 4950084 reads for set A. For each of the six samples of set B 825014 reads where randomly drawn, therefore set B in total consisted of 4950084 reads.

For performing the bioinformatic analysis of the read sets the CLC Genomics Workbench V3.6.5 (CLC bio, Denmark) was used.

The 5' primer sequences were trimmed off the reads, all erroneous nucleotides (N's) were clipped off the reads and reads below a threshold length of 20 nucleotides were excluded from further analysis.

The resulting 4940840 and 4948650 for sets A and B were used for subsequent analysis.

### a) Alignment to a reference mRNA database

The refMrna database was downloaded [1] on the 4^{th} of October 2009 from the UCSC Genome Browser webpage [6] and contains 24570 reference mRNA sequences that are based on the mouse genome assembly (mm9, NCBI built 37). In order to investigate how many of these reference mRNAs could be detected without and with segregation an alignment of read set A and read set B to these reference mRNAs was done. For both alignments the following CLC parameters were used (Add conflict annotations = No; Conflict resolution = Vote; Create Report = Yes; Create SequenceList = Yes; Match mode = random; Sequence masking = No; Similarty = 0,8; Length fraction = 0,5; Insertion cost = 3; Deletion cost = 3; Mismatch cost = 2). Set A (without segregation) detected 15652 mRNA's. An increase to 15702 detected mRNA's could be observed for data set B. As data set B contained only 6 out of 12 possible subpools this slight increase is significant.

However as the refMrna dataset contains only on the order of one transcript per known gene, an alignment of both sets to a more complete dataset that contains also more transcript variants of genes (e.g. splice variants) was carried out.

### b) Alignment to 328358 mRNA sequences

328358 GenBank mRNA sequences [5] were downloaded [2] on October 4^{th}, 2009 from the UCSC genomics browser database [6]. Applying the same CLC parameters as in a) set A and set B were aligned to these 328358 GenBank mRNA sequences. Using set A 83199 sequences could be detected and using set B 87794 sequences could be detected. This amounts to about 5% more mRNA molecules that could be detected when segregation was carried out before sequencing.

Though the observed improvement is significant, even this large mRNA databases is limited in both breadth (number of genes) and depth (transcriptvariants of a gene).

Therefore in addition alternative analyses were carried out in a genomic context.

### c) Assembly against the mouse genome

The complete reference mouse genome was downloaded [3] on the 4^{th} of October 2009 from UCSC genome browser database [6]. Alignments were made using the same CLC parameters as in a) and resulted in a genomic coverage of 0.494% for data set A and 0.561% for data set B. Therefore, set B detects about 13.5% percent more of the genome compared to set A. This translates to about 1835663 additionally mapped nucleotides. If the mean exon size for mouse is about 300-400 bases then about 4589 to 6118 additional exons can be detected.

### d) RNA-Seq analysis against the annotated mouse genome

Combining genomic and transcriptomic information a characterization of possible unknown Exons within rather narrow borders of up to 1000 bases up and downstream of known genes was performed. Here, the complete annotated reference mouse genome that was downloaded from NCBI [4] database (NCBI Build 37, mm9, C57BL/6J, July 2007) was used as a reference. An RNA-Seq analysis [7] was carried out using again the CLC Genomics Workbench. The parameter set were modified in order to include 1000 nucleotides up-and downstream of annotated gene sequences (Additional upstream bases = 1000; Addtional downstream bases = 1000; Create list of unassembled reads = Yes; Exon discovery = Yes; Maximum number of mismatches (short reads) = 2; Minimum length of putative exons = 50; Minimum number of reads = 10; Organisme type = Eukaryote; Unspecific match limit = 10; Use colorspace encoding = No; Use gene annotations = Yes; Expression value = RPKM; Minimum exon coverage fraction = 0,2; Minimum length fraction (long reads) = 0,9). Integration of data set A revealed 207 putatively novel exons of which at least 73 were uniquely detected by set A alone. Data set B improved these numbers markedly and yielded 256 putatively novel exons, at least 122 of which were discovered by B alone. Therefore segregation will reveal more novel information even in the context of known genes.

In conclusion, experiment 1 shows that segregating mRNA employing even a small matrix (12 subpools) and furthermore using only half of such a matrix (6 out of 12 subpools) the detection of mRNAs significantly improves in a genomic as well as transcriptomic context.
[1]http://hgdownload.cse.ucsc.edu/goldenPath/mm9/bigZips/refMrna .fa.gz
[2]http://hgdownload.cse.ucsc.edu/goldenPath/mm9/bigZips/mrna.fa .gz
[3]http://hgdownload.cse.ucsc.edu/goldenPath/mm9/bigZips/chromFa .tar.gz
[4]http://www.ncbi.nlm.nih.gov/.
[5] Benson, Dennis A. ; Karsch-Mizrachi, Ilene ; Lipman, David J. ; Ostell, James ; Sayers, Eric W.: GenBank. In: Nucleic Acids Res 37 (2009) Nr. Database issue, S. D26-31
[6] Kuhn, R. M. ; Karolchik, D. ; Zweig, A. S. ; Wang, T. ; Smith, K. E. ; Rosenbloom, K. R. ; Rhead, B. ; Raney, B. J. ; Pohl, A. ; Pheasant, M. ; Meyer, L. ; Hsu, F. ; Hinrichs, A. S. ; Harte, R. A. ; Giardine, B. ; Fujita, P. ; Diekhans, M. ; Dreszer, T. ; Clawson, H. ; Barber, G. P. ; Haussler, D. ; Kent, W. J.: The UCSC Genome Browser Database: update 2009. In: Nucleic Acids Res 37 (2009) Nr. Database issue, S. D755-61
[7] Mortazavi, Ali ; Williams, Brian A. ; McCue, Kenneth ; Schaeffer, Lorian ; Wold, Barbara: Mapping and quantifying mammalian transcriptomes by RNA-Seq. In: Nat Methods 5 (2008) Nr. 7, S. 621-8

### Example 2: cDNA segregation through selective precipitation and downstream NGS.

In a first step, purified mRNA of a tissue sample becomes reverse transcribed and pre-amplified. In a second step, the pre-amplified cDNA is precipitated into different fractions by increasing PEG concentrations [8]. By this means, 10 pools are prepared which contain cDNA with different solubility. The solubility is manly influenced through the length of the cDNA.

The cDNA of the 10 different sub-pools is processed separately which involves fragmentation and labeling of each sub-pool with a sub-pool specific sequence tag. All fragments are transferred to the NGS platform and sequenced, reading out in addition the tag.

The reads are segregated according to the 10 different sub-pool tags. Now, in a first assembly contigs are built by aligning reads within each subpool. In comparison, in a second assembly contigs are built neglecting the sub-pool information. More and longer contigs can be assembled using in the first assembly when contig building was done within each subpool, compared to the second assembly, where the reads where not separated into subpools.
[8] Lis, John ; Size fractionation of double-stranded DNA by precipitation with polyethylene glycol. Nucleic Acids Research, volume 2 number 3 March 1975

### Example 3: mRNA segregation through size separation and downstream NGS

10µg of mRNA of a tissue sample is electrophoretically separated on an agarose gel. After the densitometric characterization of the gel picture 12 bands are cut out. The bands hold about the same amount of mRNA. Each band is defined through one lower and one higher cut-off length according to the weight marker. The bands segregate all mRNA according to 1) 25-100bp, 2) 100-500bp, ... 12) 12000-∞bp. The mRNA is purified from the gel bands, prepared separately for NGS sequencing adding a sequence tag to each of the 12 subpools. The 12 tagged subpools are mixed in equal amounts and sequenced in one lane on an Illumina Genome Analyzer II instrument.

The NGS provides 12 times 0.8 Mio reads. Now, in a first analysis the reads are aligned to each other under guidance of the known consensus genome with the aim to construct complete transcripts. Transcripts not only have to oblige the sequence matches, in addition, each transcript must have a certain lower length and is not allowed to exceed a maximum length with respect to its band size sub-pool. In comparison, a second alignment is done neglecting the sub-pool and size information. In comparison the mean contig length of the first alignment is higher and the first alignment contains more full length sequences than the second alignment.

### Example 4: Computerized calculation of improvement.

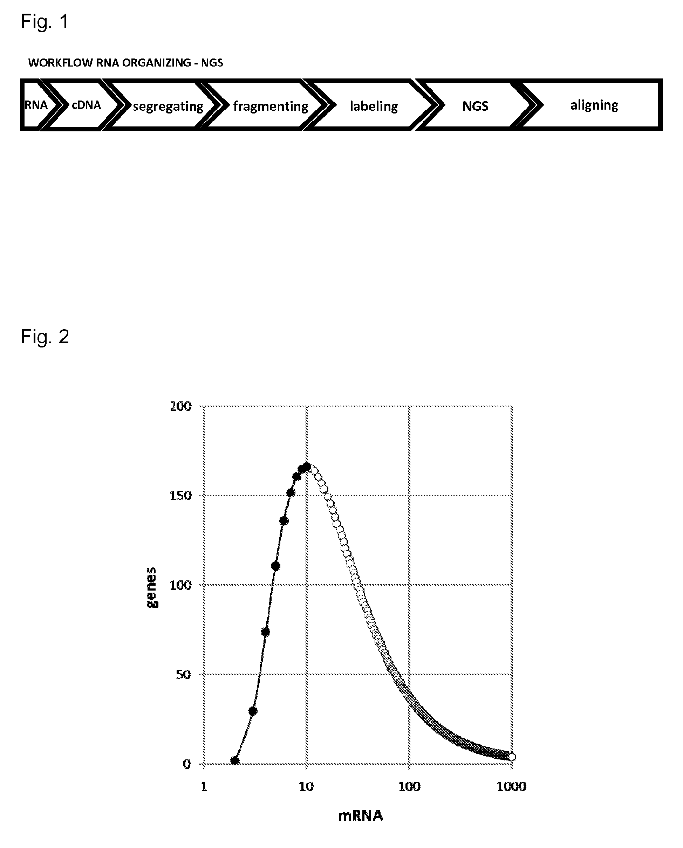

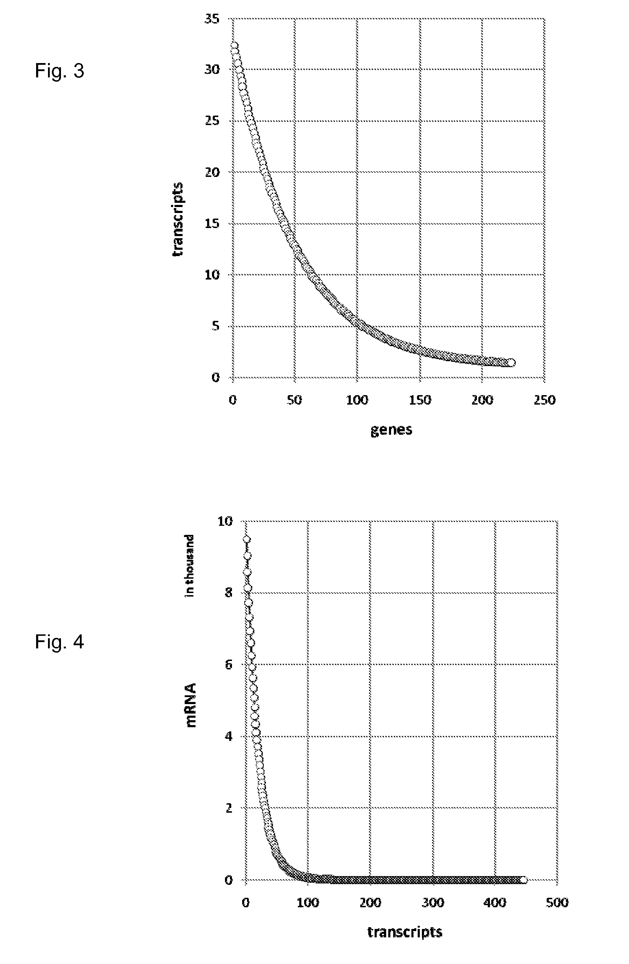

Random sequences were generated using a Random Letter Sequence Generator (http://www.dave-reed.com/Nifty/randSeq.html) and arranged in a data base, e.g because of the small size it could be done in a spreadsheet, assembling the genes of the model genome. All randomized numbers (e.g. gene and number of transcripts) were generated using a randomizer. Then, the genes were used to generate the model transcriptome according to the statistical requirements illustrated trough the graphs in fig.2 to 4. Their total number is listed in column "trans" in tab.2. To simplify matter, all transcripts are complete copies of their parental gene, so no variants are introduced yet.

For experiment 5 just 10 short genes (10 gene genome in tab. 1) have been chosen to illustrate the underlying principles in a simplistic manner.

**Table 1: Short randomized sequences used as pool model.**

| gene | length | Sequence |
|---|---|---|
| 1 | 202 | CATTACGTCCATATGAGTTCACGGTCCCTTGAACTTTTATGGTAGGTGGTAGGCTCGGCGAATC- |
| | | TAGCTTTGGAGCTTCGCCGGACTCAACAAGGTAAGGAGGAGCATCGCTCTCTCGACCACTCAA- GACGGGATATTACTTGTGTCAAGGAGATAATCGGAACTATTCTTTAGAATC- CAGCTCGCCGAAATCGTCAGGCGA |
| 2 | 242 | CTAGCTTCGGTGTCATCCCGGAAGGCCCACGTTGTGCGGCAATACTAGAGA- TAAAAGCGGCAAAGCTAACACCGAAAGCCTATACTGGCTACCCGTCTCGTTCGGTGGCAC- TAACTAGACTCCTCATCAGGCATAGGTGACCGCTCGCTCTCGTGCCAAGGTCTCCCGA- GACTTCCGAGATAGTAATAACTGAACATGGAGACCGGTATTGTTAAGCTACATCAATTGTGGGCGAAACGAAC |
| 3 | 275 | CGTTCCGGCCTGAAGCTCGGGGATCCGGCCCCCCCCTAACTTCGCTTTCTCAAACGTACAAAT- CAACCTTACTCGCATGCAGTAGATCTGCTTTGGGCCGTATCA- CACCTTCGGCTTGCCGTAAACCTGAATAGCAAATGCGGGAGG- GACTTTCCGTAATGTTGGGAATTACTTAAACACATCTTCCGGGAGCACAATTTTCCGCCACT- CAACACGGGTTTTCCTTGGTGCGTCTCATGCAATTCTTCAGTGATGGGATACTTGGCAGGGATATC |
| 4 | 297 | CAGCCCGGACGCACTGAGATGATACGTGTTGAACCGGCCTTCACTGTATATTATGCTCAC- GAGCCCTAGATTCATCAAAAAACAGGTACACTTCTCATCCTGACTATACAGCTTTCAGT- CATCCTACGATGGGAATCTAGAGCCCATAGACATATATGAGCACACTACTCTTGGTAA- CATCTCTTGTCACATACATTCGCCAATCTGAATCCTTTTCTGACAGCCAGTTCTCATGATC- CAACACTTAAGGATTTAGCATTACGGGGCGGGAGGAGAATCGAATACTCGCCCACCGTC |
| 5 | 305 | ACTGGAGAGCACCGAACATACTCCTAGCCCGGGATGACAATGTCCTAACGCCACCCAC- TAAGGGTAAGGCTCTAATTGGAAGGTAGTCCAAATACGCTCCATGAC- GAGCTTCGCTCTCAAGGCTCGCAGTCAGAACGTATCGACTATGCGACTCTAATTCCAAACCCA- GAACCTGAGCGAGGCAGTCGTTAGTTAATGACGCTTGCCGAGAGAACAGTAAAG- GAGTTCTTCGATGAGGTACTACGACATTCACATGTGTCATGGGTCGGTTAAG- CATCTGCGTGATTGATTCCGGGGGGGTGTT |
| 6 | 297 | CAGCAGGTCGCATATATCAAAAGGGAAAGCCAGCTCGCCTAGACGTCGTTCAATGGTAGG- TACTTTAATTTTTAGAGGGGCTTCCCCATGCTTTTGGAGATTGGCCTATCGGTAGTGAGGA- TACCGGCCTCCACGCTGCGTGATGAGCACAATCATTGTTCTCGGAGACGGAGGACCCGGAAGG- TAACGAGCCCAAAGGTCATTCATACCATATAGGGCGTAACCTCATTTAGCGCGACT- GACGTGCAAGGGGCATCCGACCTGCGAGGAAGGGGCCTTGGCTCTGTAGGATATAAT |
| 7 | 275 | TATCGAAAGCCCTAAGGATTTTTTTGGGGAATCGATTGTGTTAAGCAGG- GACGGCTTCAAAATTCGTCTAATAAGATTCTCTGGCCATTACCCTAACAGCGCCATACTCTA- TAGACGCACGCCTACCTTAGGCGCCTCCCGTCCCCGGATCCGAGCTCCCAAAACCCAGC- GACCTCTTCATGCTAAGGACTTCATTTGGACCCGTCAGGCACTGCTCCATGAAGAACGACAT- GAGGATTTGGAGTATTAAAGGCTTAACACTGTAGCGCCACCG |
| 8 | 242 | GTGTCGTAACTGAGCGATACAGAACGACGCTGAGTCATCGAGG- CAAATGCGTCCACCCGCACCTGCGCATCCCATACAAGGTGGCACAACTTAGTAGGACTTA- TATGCGGACTTCACCGGTACGAGAAGAGTTGAAGACTAAATTATGACGTGACAAACGAAAGAG- TAAAACAACATGCGTAGCTCTTCATGAAGCGGCAGAGCAAACCTTGAT- TAAACCCCTTGATTGGCAACACTACACG |
| 9 | 202 | CGGTTACCCGGCGTTAGGCCTATGTACCGCCCGACGTACTTGCTAGGGGTCATACTACCGAC- GATCCCTGCTAACAAAGAACAGTACCGGCTTTCCTTAACTACTCAGTGCTACTAAAACTAG- |
| | | CATGAGGGTTGAGATCATCTCATCCAGTTGGGTCCAGCGCATGAT- TAATTGCTTTACTCGCACTTTAATTCGGCTTCTA |
| 10 | 160 | GGAGGCACGACGAGTATCTAGTGTCTGCACGGGACTCCGGAGGACATTCCCTACAAGT- TACCGGCGTCAGTAGCAGCAAGACTGGTCTGTCTACCCCTGCCTGA- CAAAGTCTTTCTTGGATTTCGGACCGAAACTCGGCCCAACATGCCATTGGCCATAT |

First, the transcripts were ordered into 16 (4x4) different pools according to their terminal bases (tab. 3).

Because one particular transcriptome (all reads align to the blue print) is selected and any reading errors are excluded, a simple alignment algorithm (simple search function which provides the number of sequence matches) could be used to probe the genome/transcriptome. It selects all reads that have a perfect k-mer match to the reference sequence (transcriptome). So, 24 permutations of 4bp fragments (without any base repeats like AATG) were taken and aligned, once against the entire model genome/transcriptome (tab. 2) and once against the segregated genome/transcriptome (tab. 3). The number of unique hits is shown in both tables in the right column.

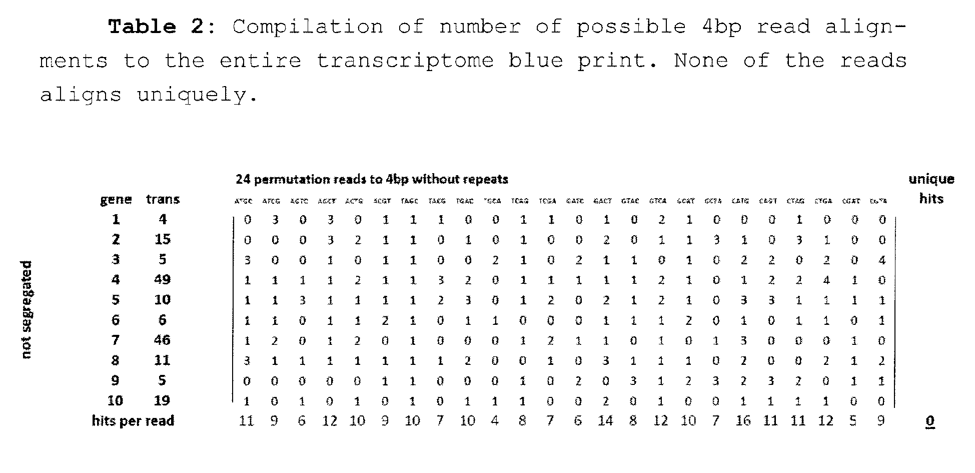

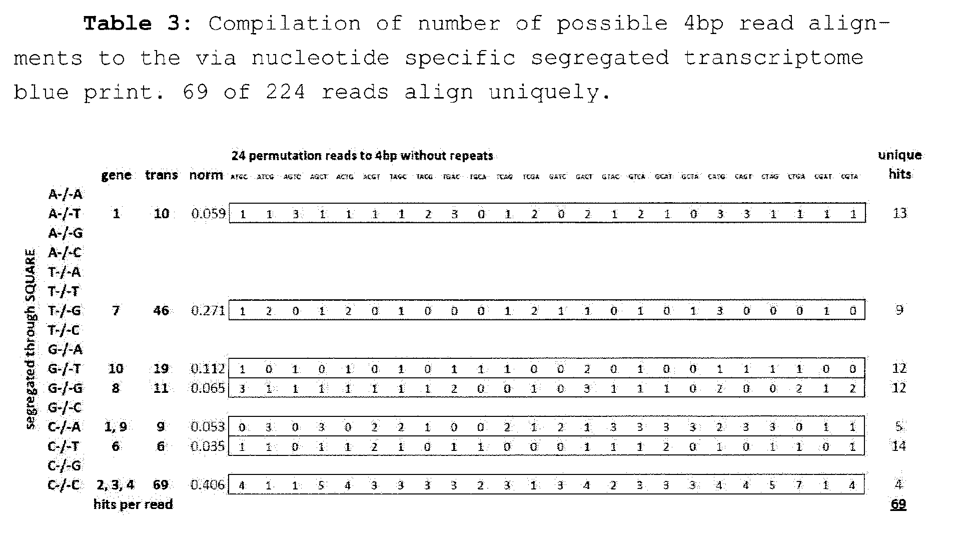

This example shows, that
i) none of the 24 probed reads gave one unique hit when trying to align reads to the entire genome/transcriptome. The number of total hits was 224. The most unique read aligned matched 4 different genes/transcripts.
ii) After segregation into 7 sub-pools, here according to the molecule ends, 69 (31%) of the reads could already be aligned uniquely.

Even without having a blue print the same principle applies. In the first case none of the investigated reads will belong to a unique position in the pool, whereas 31% of the reads will have one unique position in their host sub-pool. The relative value regarding the alignment within the pool of transcripts is given by the number in the column "norm" in tab. 3. For example, the 4 unique hits in pool C-/-C containing the highly abundant transcripts of genes 20, 30 and 40 do uniquely identify close to 40 percent of all transcripts.

### REFERENCES:

Maruyama, K. and Sugano, S. (1994) Oligo-capping: a simple method to replace the cap structure of eukaryotic mRNAs with oligoribonucleotides. Gene, 138, 171 - 174.
Shiraki, T., Kondo, S., Katayama, S., Waki, K., Kasukawa, T., Kawaji, H., Kodzius, R., Watahiki, A., Nakamura, M., Arakawa, T., Fukuda, S., Sasaki, D., Podhajska, A., Harbers, M., Kawai, J., Carninci, P. and Hayashizaki, Y. (2003) Cap analysis gene expression for high-throughput analysis of transcriptional starting point and identification of promoter usage. Proc Natl Acad Sci U S A, 100, 15776-81.

## Claims

1. Method of ordering nucleic acid molecule fragment sequences derived from a pool of potentially diverse RNA molecules comprising
• optionally reverse transcribing the RNA molecules to provide a pool of cDNA molecules,
• segregating nucleic acids from said template RNA or cDNA pool, selecting for potentially different templates with a distinctive nucleic acid feature shared by the segregated templates, thereby providing at least a first subpool of nucleic acids,
• optionally once or more further segregating nucleic acids from said template RNA or cDNA, selectively segregating nucleic acids with a different distinctive nucleic acid feature, thereby providing one or more further subpool(s) of nucleic acids,
• generating fragments of said segregated nucleic acid molecules by fragmenting or obtaining fragment copies of said segregated nucleic acid molecules, wherein the fragments of each subpool or combined subpools remain separable from fragments of other subpools or other combined subpools by physically separating the subpools or by attaching a label to the fragments of the subpools, with the label identifying a subpool, or determining a partial sequence of said segregated nucleic acid molecule and preferably aligning at least two sequences or partial sequences to a joined sequence.

2. The method of claim 1 further comprising determining the sequence or partial sequence of the fragments of the first subpool and optionally further subpools, preferably wherein a partial sequence of at least 10, in particular preferred at least 18, even more preferred at least 25, nucleotides is determined.

3. The method of claim 1 or 2, **characterized in that** the RNA molecules are of a biological sample, preferably of a virus, prokaryote or eukaryote.

4. The method of any one of claims 1 to 3, **characterized in that** fragmenting the segregated nucleic acid molecules comprises random fragmenting, preferably by physical means, in particular preferred by shearing, sonication or elevated temperatures.

5. The method of any one of claims 1 to 4, **characterized in that** the fragments consist of 10 to 10000 nucleotides, preferably of 25 to 500 nucleotides.

6. The method of any one of claims 1 to 5, **characterized in that** the nucleic acid feature is a given nucleotide type, preferably selected from any one of A, T, U, G, C, at a certain position in the nucleic acid molecule, preferably the position being within 100 nucleotides from either the 5' or 3' terminus of the nucleic acid molecule.

7. The method of claim 6, **characterized in that** the nucleic acids are selected for common nucleotides within the 10 nucleotides next to the 5' and/or 3' terminus, preferably for one or more common 5' and/or 3' terminal nucleotide types.

8. The method of any one of claims 1 to 7, **characterized in that** said RNA molecule is a full length RNA and/or the segregated nucleic acid molecule comprises the sequence of the full length or complete cDNA or RNA.

9. The method of claim 2, **characterized in that** sequence determinations comprises determining the sequence of at least 5, preferably at least 8, nucleotides from the fragment, in particular from either its 5' or the 3' end, even more preferred determining the full sequence of the fragment.

10. The method of any one of claims 1 to 9, **characterized in that** the nucleic acids are divided into subpools wherein at least 10% of all subpools comprise the average amount of nucleic acids of all subpools +/-50%.

11. The method of any one of claims 1 to 10, **characterized in that** the nucleic acids are divided into subpools wherein at least 10% of the subpools contain 2 or less nucleic acids, preferably 1 nucleic acid.

12. The method of any one of claims 1 to 11, **characterized in that** segregating nucleic acids comprises specifically amplifying nucleic acids from said template pool.

13. The method of claims 12, **characterized in that** amplification is performed by nucleotide extension from a primer, preferably by PCR, in particular preferred wherein the amplification is performed by using primers which select for at least one, preferably at least two, in particular at least two adjacent, different nucleotides after an unspecific primer portion whereby nucleic acid molecules are amplified which comprise the selected nucleotide as the nucleic acid feature specific for a subpool.

14. The method of any one of claims 1 to 13, **characterized by** attaching a subpool-specific label to the fragments.

15. The method of claim 14, **characterized in that** the subpool-specific label is one or more nucleotides, which are preferably co-determined during sequence determination as defined in claim 2.

16. The method of any one of claims 1 to 15, further comprising amplifying the nucleic acid molecules, preferably after segregation, prior to determining the sequence, in particular preferred wherein said amplification is by PCR and at least one nucleotide molecule is amplified to the saturation phase of the PCR, in particular preferred at least 10% of the different nucleotide molecules are amplified to the saturation phase of the PCR.

17. The method of any one of claims 1 to 16, **characterized in that** subpools with high abundant nucleic acid molecules are excluded from sequence determination, wherein subpools with high abundant nucleic acids molecules are subpools comprising more than 1000% nucleic acid molecules above the average amount of all subpools.

18. The method of any one of claims 1 to 17, **characterized in that** during segregation of the nucleic acid one selected strand is segregated or one selected strand is labeled, wherein preferably the fragments of the seleceted strand are also labeled.


SEQUENCE LISTING

<110>  Lexogen GmbH

<120>  RNA analytics method

<130>  r54669

<160>  10

<170>  PatentIn version 3.5

<210>  1
<211>  202
<212>  DNA
<213>  Artificial Sequence

<220>
<223>  random sequence

<400>  1
cattacgtcc atatgagttc acggtccctt gaacttttat ggtaggtggt aggctcggcg      60

aatctagctt tggagcttcg ccggactcaa caaggtaagg aggagcatcg ctctctcgac     120

cactcaagac gggatattac ttgtgtcaag gagataatcg gaactattct ttagaatcca     180

gctcgccgaa atcgtcaggc ga                                              202

<210>  2
<211>  242
<212>  DNA
<213>  Artificial Sequence

<220>
<223>  random sequence

<400>  2
ctagcttcgg tgtcatcccg gaaggcccac gttgtgcggc aatactagag ataaaagcgg      60

caaagctaac accgaaagcc tatactggct acccgtctcg ttcggtggca ctaactagac     120

tcctcatcag gcataggtga ccgctcgctc tcgtgccaag gtctcccgag acttccgaga     180

tagtaataac tgaacatgga gaccggtatt gttaagctac atcaattgtg ggcgaaacga     240

ac                                                                    242

<210>  3
<211>  275
<212>  DNA
<213>  Artificial Sequence

<220>
<223>  random sequence

<400>  3
cgttccggcc tgaagctcgg ggatccggcc cccccctaac ttcgctttct caaacgtaca      60

aatcaacctt actcgcatgc agtagatctg ctttgggccg tatcacacct tcggcttgcc     120

gtaaacctga atagcaaatg cgggagggac tttccgtaat gttgggaatt acttaaacac     180

atcttccggg agcacaattt tccgccactc aacacgggtt ttccttggtg cgtctcatgc     240

aattcttcag tgatgggata cttggcaggg atatc                                275

<210>  4
<211>  297
<212>  DNA
<213>  Artificial Sequence

<220>
<223>  random sequence

<400>  4
cagcccggac gcactgagat gatacgtgtt gaaccggcct tcactgtata ttatgctcac      60

gagccctaga ttcatcaaaa aacaggtaca cttctcatcc tgactataca gctttcagtc     120

atcctacgat gggaatctag agcccataga catatatgag cacactactc ttggtaacat     180

ctcttgtcac atacattcgc caatctgaat ccttttctga cagccagttc tcatgatcca     240

acacttaagg atttagcatt acggggcggg aggagaatcg aatactcgcc caccgtc        297

<210>  5
<211>  304
<212>  DNA
<213>  Artificial Sequence

<220>
<223>  random sequence

<400>  5
actggagagc accgaacata ctcctagccc gggatgacaa tgtcctaacg ccacccacta      60

agggtaaggc tctaattgga aggtagtcca aatacgctcc atgacgagct tcgctctcaa     120

ggctcgcagt cagaacgtat cgactatgcg actctaattc caaacccaga acctgagcga     180

ggcagtcgtt agttaatgac gcttgccgag agaacagtaa aggagttctt cgatgaggta     240

ctacgacatt cacatgtgtc atgggtcggt taagcatctg cgtgattgat tccggggggg     300

tgtt                                                                  304

<210>  6
<211>  297
<212>  DNA
<213>  Artificial Sequence

<220>
<223>  random sequence

<400>  6
cagcaggtcg catatatcaa aagggaaagc cagctcgcct agacgtcgtt caatggtagg      60

tactttaatt tttagagggg cttccccatg cttttggaga ttggcctatc ggtagtgagg     120

ataccggcct ccacgctgcg tgatgagcac aatcattgtt ctcggagacg gaggacccgg     180

aaggtaacga gcccaaaggt cattcatacc atatagggcg taacctcatt tagcgcgact     240

gacgtgcaag gggcatccga cctgcgagga aggggccttg gctctgtagg atataat        297

<210>  7
<211>  275
<212>  DNA
<213>  Artificial Sequence

<220>
<223>  random sequence

<400>  7
tatcgaaagc cctaaggatt ttttttgggg aatcgattgt gttaagcagg gacggcttca      60

aaattcgtct aataagattc tctggccatt accctaacag cgccatactc tatagacgca     120

cgcctacctt aggcgcctcc cgtccccgga tccgagctcc caaaacccag cgacctcttc     180

atgctaagga cttcatttgg acccgtcagg cactgctcca tgaagaacga catgaggatt     240

tggagtatta aaggcttaac actgtagcgc caccg                                275


<210>  8
<211>  242
<212>  DNA
<213>  Artificial Sequence

<220>
<223>  random sequence

<400>  8
gtgtcgtaac tgagcgatac agaacgacgc tgagtcatcg aggcaaatgc gtccacccgc      60

acctgcgcat cccatacaag gtggcacaac ttagtaggac ttatatgcgg acttcaccgg     120

tacgagaaga gttgaagact aaattatgac gtgacaaacg aaagagtaaa acaacatgcg     180

tagctcttca tgaagcggca gagcaaacct tgattaaacc ccttgattgg caacactaca     240

cg                                                                    242


<210>  9
<211>  202
<212>  DNA
<213>  Artificial Sequence

<220>
<223>  random sequence

<400>  9
cggttacccg gcgttaggcc tatgtaccgc ccgacgtact tgctaggggt catactaccg      60

acgatccctg ctaacaaaga acagtaccgg ctttccttaa ctactcagtg ctactaaaac     120

tagcatgagg gttgagatca tctcatccag ttgggtccag cgcatgatta attgctttac     180

tcgcacttta attcggcttc ta                                              202


<210>  10
<211>  160
<212>  DNA
<213>  Artificial Sequence

<220>
<223>  random sequence

<400>  10
ggaggcacga cgagtatcta gtgtctgcac gggactccgg aggacattcc ctacaagtta      60

ccggcgtcag tagcagcaag actggtctgt ctacccctgc ctgacaaagt ctttcttgga     120

tttcggaccg aaactcggcc caacatgcca ttggccatat                           160